# EUROPEAN PATENT APPLICATION

(11) **EP 2 223 608 A1**
(43) Date of publication of application: **01.09.2010**
(21) Application number: 07817125.3
(22) Date of filing: 22.10.2007
(51) Int. Cl.: A23J 3/14, C11B 1/06, C11B 1/10, A23N 5/08, A23N 12/02

(54) **ABELMOSCHUS MANIHOT (LINN.) MEDIUS KERNEL PRODUCT FOR NATURAL NUTRITIONAL EDIBLE AND PHARMACEUTICAL RAW MATERIAL**

(71) Applicant: Beijing Dongsheng Agricultural Technology Development (Group) Co., Ltd, Beijing 101105 (CN)
(72) Inventor: Wang Zhiyuan, Hebei 054200 (CN); Wang Shaozhang, Hebei 054200 (CN)
(74) Representative: Nevant, Marc
(86) International application number: PCT/CN2007/070934
(87) International publication number: WO 2009/052681

(57) **Abstract**

An Abelmoschus manihot (Linn.) medius kernel product for natural nutritional edible and pharmaceutical raw material. It is prepared by removing impurity and then cracking the shell of Abelmoschus manihot (Linn.) medius seed; and removing the seed shell to separate the particle powder of the Abelmoschus manihot (Linn.) medius seed, and degreasing the particle powder of the Abelmoschus manihot (Linn.) medius seed. It is used as the raw materials of edible product or biomedical product.

## Description

### FIELD OF THE INVENTION

The present invention belongs to the plant resources development field and relates to a kind of raw materials of edible product or biochemical product.

### BACKGROUND OF THE INVENTION

Abelmoschus manihot (Linn.) Medicus is a kind of herbs belonging to Abelmoschus of Malvaceae. Its code in China National Standards GB/T14467-93 implemented on January 1, 1994 is 6000010003. In the document with the publication number CN101002536A, Hibiscus manihot is an optimally selected population of Abelmoschus manihot (Linn.) Medicus. Many documents and published papers on the medicinal development of the plant all claim that it is nontoxic. The inventor investigateed the people who ate this plant in the society and there were several thousand people who ate entire fresh twig and ground powder of the entire plant. Esculentus(Linn.)Moench of the same genus with Abelmoschus manihot (Linn.) Medicus has the China National Standard code 6000010002, whose kernel development has no application scheme. There are documents referring to the practical application cases. For example, Food and Agriculture Organization of the United Nations mentioned in *Tropical Plant* that "... it is planted as vegetable and its green and long pods are harvested. The unripe pods contain a kind of adherent substances. The seeds of the ripe pods are sometimes collected as chicken feed, which can supplement protein required by monogastric animals. It is said that the seeds contain cottonseed phenol". There has been no any second document with Abelmoschus manihot (Linn.) medius kernel product as the theme besides those proposed by inventor of the present invention.

### SUMMARY OF THE INVENTION

The objective of the present invention is to provide a kind of high protein materials and kernel fat for edible or bio-medical raw materials. The protein content of the high protein materials is 32.5% and the quality of the protein is as good as soy protein, but they don't contain harmful biological enzymes which exist in the soy protein, such as the harmful saponins and so on. The kernel fat is rich in natural Vitamin E, oleic acid and linoleic acid, whose quality is superior to sallow thorn oil highly recommended by NASA.

The design of the present invention is that it is prepared by removing impurity and then cracking the shell of Abelmoschus manihot (Linn.) medius seed; and removing the seed shell to separate the particle powder of the Abelmoschus manihot (Linn.) medius seed, and degreasing the particle powder of the Abelmoschus manihot (Linn.) medius seed. Specifically speaking:
An Abelmoschus manihot (Linn.) medius kernel product prepared by processing Abelmoschus manihot (Linn.) medius kernel, wherein it is **characterized in that** it is prepared by removing impurity and then cracking the shell of Abelmoschus manihot (Linn.) medius seed; and removing the seed shell to separate the particle powder of the Abelmoschus manihot (Linn.) medius seed, and degreasing the particle powder of the Abelmoschus manihot (Linn.) medius seed.

Remove impurity, crack the shell of Abelmoschus manihot (Linn.) medius seed, remove the seed shell to separate the particle powder of the kernel and then mechanically degrease the particle powder of Abelmoschus manihot (Linn.) medius kernel. The Abelmoschus manihot (Linn.) medius kernel after degreasing is called the oily pure product, namely the oily pure product of Abelmoschus manihot (Linn.) medius kernel. Remove the impurity of the oiliness from degreasing the particle powder of the Abelmoschus manihot (Linn.) medius seed and the product is called the fat product of the belmoschus manihot (Linn.) medius kernel.

Extract and degrease the particle powder of the Abelmoschus manihot (Linn.) medius kernel with seed shell removed or the oily pure product of the Abelmoschus manihot (Linn.) medius kernel mechanically degreased and the degreased Abelmoschus manihot (Linn.) medius kernel is called the pure product, namely pure product of the Abelmoschus manihot (Linn.) medius kernel. Remove the impurity of the fat from decreasing and the product is called the fat product of the Abelmoschus manihot (Linn.) medius kernel.

Determine the water content of the oily pure product of the Abelmoschus manihot (Linn.) medius kernel which is degreased mechanically and dry and evaporate the water content at the temperature below 180 degrees centigrade, so as to control the water content below 16%.

Determine the extraction medium content in the pure product of the Abelmoschus manihot (Linn.) medius kernel degreased by extraction, remove the extraction medium by heating at the temperature below 120 degrees centigrade and evaporate the water content, so as to control the water content below 16%. The extraction medium content is controlled within 50mg/kg according to national standard.

Disposal of the kernel fat from degreasing the Abelmoschus manihot (Linn.) medius kernel: Remove the impurities of crude oil made from press degreasing or extraction degreasing by static sedimentation first, then separating the fat from the sediments and finally treat it routinely.

The invention product is used as edible or bio-medical raw materials.

The optimal selection of above mentioned Abelmoschus manihot (Linn.) medius is the Hibiscus manihot population of Abelmoschus manihot (Linn.) Medicus, mentioned in the BACKGROUND OF THE INVENTION.

The specific procedures are:
1. Clean the Abelmoschus manihot (Linn.) Medicus seeds and remove dust, sand and plant fragments, etc. Use the air separation and screening equipment for cleaning the grains to clean the seeds and adopt the operation for cleaning wheat, barley and other grains. The cleaned Abelmoschus manihot (Linn.) Medicus seeds are called pure seeds.
II. Crack the shells of the cleaned Abelmoschus manihot (Linn.) Medicus pure seeds. The weight, thickness and robustness of the Abelmoschus manihot (Linn.) Medicus seed coat is greater than all the grains and can be compared with the cotton seed shell. However, there are kernel membranes between the kernels in the cotton seed shells which are easy to peel off, just like the buckwheat in the grains, while the Abelmoschus manihot (Linn.) Medicus seed shell is closely combined with the kernel and it is hard to peel off with the shucking equipment, so corresponding shell cracking treatment must be carried out. There are three methods to crack the shells: the first is to crack the shell with the rolling equipment for grains; the second is to hit the shell with the breaking equipment; the third is to crack the shell with swelling equipments. The Abelmoschus manihot (Linn.) Medicus seed after cracking is called pre-material.
III. Remove the shell of the cracked Abelmoschus manihot (Linn.) Medicus seed, namely the pre-material. The property of the Abelmoschus manihot (Linn.) Medicus seed shell is even tougher than that of the cotton seed shell and it is hard to grind it just like the husks of wheat and millet for edible. If not removed well, besides the product application, the degreasing process will also be influenced directly, so corresponding method shall be taken to remove the Abelmoschus manihot (Linn.) Medicus seed shell. There are three methods to remove the shell: the first is to sieve and separate with the normal sieving method for grains; the second is air flotation method to remove wheat bran for making flour; and the third is to peel off the shell of the swelled seed with the method for processing the pine nuts of the pines. The particle powder of the shelled Abelmoschus manihot (Linn.) medius kernel is called pure material.
IV Degrease the particle powder of the shelled Abelmoschus manihot (Linn.) medius kernel, namely the pure materials. There is more than 15% fat in the pure materials, which is very easy to oxidize without degreasing, so it is difficult to store and transport the products and the flavor and protein content control will be influenced. Therefore, degrease the pure materials timely after there are obtained. For the production technique design of non-automatic procedure and continuous production, the degreasing time after getting the pure materials shall be less than 21 days under the conventional condition. There are two degreasing methods. One is direct degreasing with the mechanical pressing or screw extrusion the same as obtaining the cotton seed oil, the particle powder of the decreased Abelmoschus manihot (Linn.) medius seed is called the oily pure material, namely the oily pure material product of the Abelmoschus manihot (Linn.) medius, and the fat from degreasing is called the Abelmoschus manihot (Linn.) medius kernel fat product. The other is to extract and degrease the pure materials or the oily pure materials after press degreasing the same as the method for extracting soybean oil, the particle powder of the degreased Abelmoschus manihot (Linn.) medius kernel is called the pure material product of Abelmoschus manihot (Linn.) medius kernel and the fat from degreasing is called the Abelmoschus manihot (Linn.) medius kernel fat product.
V. During the completion process of the present invention product, three substances are got from the above three-step technique, oily pure materials product, pure materials product and kernel fat. If the three substances are not suitably treated, it is hard to control and maintain their quality and it is difficult to meet the standard and apply during the storage, transportation and application process. Therefore, corresponding disposal must be carried out on them to make them become the invention products. There are three disposals. The first is to determine the water content of the oily pure material seed powder, dry and evaporate the water at the temperature below 180 degrees centigrade, so as to control the water content below 16%. The second is to determine the content of the extraction medium in the pure materials, heat and remove the extraction medium at a temperature below 120 degrees centigrade and evaporate the water at the same time to control the water content below 16% and the extraction medium content below 50mg/kg. The third is disposal of the kernel fat. Remove the impurity of the crude oil from press degreasing or extraction decreasing production by adopting static sedimentation first, then separating the sediments and finally carrying out the routine treatment.
VI.The Abelmoschus manihot (Linn.) medius seed powder products treated by the above five processes are called products of the present invention. The above mentioned oily pure materials are called oily pure material product of the Abelmoschus manihot (Linn.) medius kernel, the pure materials are called Abelmoschus manihot (Linn.) medius kernel pure material products, kernel fat is called Abelmoschus manihot (Linn.) medius kernel fat product. The three products are used for edible or pharmaceutical raw materials as the filling respectively.

The optimal selection of above mentioned Abelmoschus manihot (Linn.) medius is the Hibiscus manihot population of Abelmoschus manihot (Linn.) Medicus mentioned in the BACKGROUND OF THE INVENTION.

### BENEFICIAL EFFECTS

As far as the present biological resource development information analysis is concerned, the present invention product ranks highest concerning the plant protein and is in the front rank concerning the natural unsaponifiable matter, including the types, cleanness and content of natural Vitamin E. And the costs is 3% of the comparable products.

All the quantitative indexes and utilizing effects of the present invention product is superior to the development products of all the traditional food resources. For example,

Amino acid analysis of the Abelmoschus manihot (Linn.) Medicus kernel pure materials: Code: Bookman 121MB, sample amount: 80.43mg, dilution volume: 25ml.

| aa | Conc | Mg/gSam ple | g/16gN | | aa | Conc | Mg/gSam ple | g/16g N |
|---|---|---|---|---|---|---|---|---|
| Asp | 522.14 | 16.444 | 11.0 | | Thr | 202.64 | 5.704 | 3.9 |
| Ser | 264.35 | 9.052 | 6.0 | | Glu | 250.15 | 29.551 | 19.7 |
| Pro | 241.57 | 5.822 | 4.6 | | Gly | 551.2 | 9.864 | 6.6 |
| Ala | 332.12 | 8.961 | 5.9 | | Cys | 31.292 | | |
| Val | 277.24 | 7.673 | 5.1 | | Met | 78.942 | 2.606 | 1.7 |
| Ilou | 313.12 | 6.071 | 4.1 | | Leu | 409.81 | 12.20 | 8.1 |
| Tyr | 121.16 | 5.186 | 3.5 | | phe | 187.86 | 2.332 | 1.6 |
| Lys | 235.66 | 8.830 | 6.1 | | His | 121.34 | 4.448 | 3.0 |
| Trp | | | 1.4 | | Arg | 363.53 | 15.169 | 10.4 |

Grade the indispensable amino acids of the above kernel pure materials with FAO amino acid mode as the standard

| | FAO (g/16g N) | Abelmoschus manihot (Linn.) Medicus kernel product |
|---|---|---|
| Isoleucine | 4.0 | 4.1 |
| Leucine | 7.0 | 8.1 |
| Lysine | 5.5 | 6.1 |
| Methionine + HCY | 3.5 | 3.7 |
| Phenylalanine + tyrosine | 6.0 | 10. 1 |
| Threonine | 4.0 | 3.9 |
| Tryptophan | 1.0 | 1. 4 |
| Valine | 5.0 | 5. 1 |

The above grading finds that amino acid grades of the Abelmoschus manihot (Linn.) Medicus kernel pure materials are all relatively high.

Compare unsaturated fatty acid and VE between the Abelmoschus manihot (Linn.) Medicus kernel fat product and sallow thorn oil

| | Abelmoschus manihot (Linn.) Medicus kernel fat product | Chinese sallow thorn oil |
|---|---|---|
| Oleic acid | 33. 26% | 24.4% |
| Linoleic acid | 46.78% | 8. 2% |
| E Vitamin E | 1500mg/kg | 1319mg/kg |

It is found that oleic acid, linoleic acid and Vitamin E indexes of the Abelmoschus manihot (Linn.) Medicus kernel fat products are all superior to those of sallow thorn oil.

Harmful element determination of the Abelmoschus manihot (Linn.) Medicus kernel fat products

Cu 0.08mg/ml Pb 0.03mg/ml As 0.009mg/ml Hg 0.003mg/ml

They are all far smaller than the edible vegetable oil standard of many countries.

The gossypol content of the Abelmoschus manihot (Linn.) Medicus kernel is smaller than 20ppm, which is far smaller than the 200ppm standard in many countries.

As agricultural processed products, the present invention products are used for edible or bio-medicinal raw materials.

### DETAILED DESCRIPTION OF THE INVENTION

Embodiment 1: 503kg Hibiscus manihot seed of the Abelmoschus manihot (Linn.) Medicus population. First use a vibrating sieve to remove the big and small light impurities and then adopt blower type raw material cleaning machine to remove the dust, sand and other impurities to get 500kg pure seeds. Put the pure seeds in knife board type pine nut peeling machine to get the pre-materials and use the vibrating sieve to thoroughly remove the seed shell to get 400kg pure materials. Then put the pure materials into the pressing type oil mill for degreasing and get 360kg oily pure materials product, namely Abelmoschus manihot (Linn.) Medicus kernel oily pure materials product, and 40kg kernel fat. Dehydrate the 40kg kernel fat and remove the impurities to get 38kg Abelmoschus manihot (Linn.) Medicus kernel fat product. Embodiment 2: 1007kg Hibiscus manihot seed of the Abelmoschus manihot (Linn.) Medicus population. First use a vibrating sieve to remove the big and small light impurities and then adopt blower type raw material cleaning machine to remove the dust, sand and other impurities to get 1000kg pure seeds. Put the pure seeds into rolling type peeling machine (comprising vibrating sieves and air separation systems) to directly get 800kg pure materials. Then use a screw-type continuous oil mill for degreasing and get 720kg oily pure materials and 80kg kernel fat with impurities. Separate the kernel fat with impurities through static sedimentation and get 76kg Abelmoschus manihot Linn.) Mdicus kernel fat products. Extract and separate the oily pure materials with No.2 extraction agent on the market by the operation same as extracting oil from soybean cakes and get 43kg Abelmoschus manihot (Linn.) Medicus kernel fat products and 657kg kernel pure materials to be treated. Desolventize the kernel pure materials to be treated at the temperature below 80 degrees centigrade to get 661kg kernel pure materials to be dehydrated and then dehydrate them at a temperature below 100 degrees centigrade to get 657kg Abelmoschus manihot Linn.) Mdicus kernel pure materials product.

## Claims

1. An Abelmoschus manihot (Linn.) medius kernel product processed from the Abelmoschus manihot (Linn.) medius kernel, wherein it is **characterized in that** it is prepared by removing impurity and then cracking the shell of the Abelmoschus manihot (Linn.) medius seed; and removing the seed shell to separate out the particle powder of the Abelmoschus manihot (Linn.) medius seed, and degreasing the particle powder of the Abelmoschus manihot (Linn.) medius seed.

2. The product according to Claim 1, wherein the degreased Abelmoschus manihot (Linn.) medius kernel prepared by removing impurity, cracking the shell of the Abelmoschus manihot (Linn.) medius seed, removing the seed shell to separate the kernel particle powder and degreasing the particle powder of the Abelmoschus manihot (Linn.) medius kernel by mechanical oil making is called the oily pure materials, namely the oily pure material product of the Abelmoschus manihot (Linn.) medius kernel, and the product prepared by removing the impurity of the oiliness got from degreasing the particle powder of Abelmoschus manihot (Linn.) medius seed is called the Abelmoschus manihot (Linn.) medius kernel fat product.

3. he product according to Claim 1, wherein the degreased Abelmoschus manihot (Linn.) mediums kernel prepared by extracting and degreasing the particle powder of the Abelmoschus manihot (Linn.) mediums kernel after seed shell removing or the oily pure materials of the Abelmoschus manihot (Linn.) medius kernel, namely the oily pure material products of the Abelmoschus manihot (Linn.) medius kernel, is called the pure material, namely the pure material product of the Abelmoschus manihot (Linn.) medius kernel, and the product prepared by removing the impurity of the fat got from degreasing is called the Abelmoschus manihot (Linn.) medius kernel fat product.

4. The product according to Claim I, wherein water content determination is conducted on the oily pure material product of the Abelmoschus manihot (Linn.) medius kernel degreased mechanically and the water is dried and evaporated at a temperature below 180 degrees centigrade, so as to control the water content below 16%.

5. The product according to Claim 1, wherein extraction medium content determination is conducted on the Abelmoschus manihot (Linn.) medius kernel pure materials degreased with the extraction method, extraction medium is removed by heating at the temperature below 120 degrees centigrade and the water is evaporated at the same time, so as to control the water content below 16%. The content of the extraction medium is controlled within the national standard of 50mg/kg.

6. The product according to Claim 1, wherein the disposal of the kernel fat got from decreasing the Abelmoschus manihot (Linn.) medius kernel is to remove the impurity of the crude oil made from pressing degreasing or extraction decreasing by firstly adopting static precipitation, then separating the fat from the sediments and finally conducting routine treatment.

7. The product according to Claim 1, wherein it is used for edible or bio-medicinal raw materials.

## Amended claims

### Amended claims under Art. 19.1 PCT

1. A natural complete protein processed from the Abelmoschus manihot (Linn.) medius kernel, wherein it is **characterized in that** it is prepared by removing impurity and then cracking the shell of the Abelmoschus manihot (Linn.) medius seed; and removing the seed shell to separate out the particle powder of the Abelmoschus manihot (Linn.) medius seed, and degreasing the particle powder of the Abelmoschus manihot (Linn.) medius seed.

2. The product according to Claim 1, wherein the degreased Abelmoschus manihot (Linn.) medius kernel prepared by removing impurity, cracking the shell of the Abelmoschus manihot (Linn.) medius seed, removing the seed shell to separate the kernel particle powder and degreasing the particle powder of the Abelmoschus manihot (Linn.) medius kernel by mechanical oil making is called the oily pure materials, namely the oily pure material product of the Abelmoschus manihot (Linn.) mediums kernel, and the product prepared by removing the impurity of the oiliness got from degreasing the particle powder of Abelmoschus manihot (Linn.) medius seed is called the Abelmoschus manihot (Linn.) medius kernel fat product.

3. The product according to Claim 1, wherein the degreased Abelmoschus manihot (Linn.) medius kernel prepared by extracting and degreasing the particle powder of the Abelmoschus manihot (Linn.) medius kernel after seed shell removing or the oily pure materials of the Abelmoschus manihot (Linn.) medius kernel, namely the oily pure material products of the Abelmoschus manihot (Linn.) medius kernel, is called the pure material, namely the pure material product of the Abelmoschus manihot (Linn.) medius kernel, and the product prepared by removing the impurity of the fat
got from degreasing is called the Abelmoschus manihot (Linn.) medius kernel fat product.

4. The product according to Claim 1, wherein water content determination is conducted on the oily pure material product of the Abelmoschus manihot (Linn.) medius kernel degreased mechanically and the water is dried and evaporated at a temperature below 180 degrees centigrade, so as to control the water content below 16%.

5. The product according to Claim 1, wherein extraction medium content determination is conducted on the Abelmoschus manihot (Linn.) medius kernel pure materials degreased with the extraction method, extraction medium is removed by heating at the temperature below 120 degrees centigrade and the water is evaporated at the same time, so as to control the water content below 16%. The content of the extraction medium is controlled within the national standard of 50mg/kg.

6. The product according to Claim 1, wherein the disposal of the kernel fat got from degreasing the Abelmoschus manihot (Linn.) medius kernel is to remove the impurity of the crude oil made from pressing degreasing or extraction decreasing by firstly adopting static precipitation, then separating the fat from the sediments and finally conducting routine treatment.

7. The product according to Claim 1, wherein it is used for edible or bio-medicinal raw material.

The product in the comparison document is liquid oil and what the present invention applies for is the solid complete protein. Though the raw materials of the two products are the same and the processing techniques have overlap, in order to reach different product targets, there are key differences in the technology. For example, concerning the treatment of the Abelmoschus manihot (Linn.) medius seed, the comparison document uses the one-step technique of directly peeling and the present invention adopts the twa-step technique of cracking the shell first and then the separating. As the inventor of the comparison document is of the same inventor team with the present invention applicant, they both know well the essence of the above said difference. The present invention applies in the field of traditional herbs resources development. Concerning the edible or bio-medicinal raw materials industry, the essence of its novelty and creativity is to provide different conceptual products with the grade higher than the comparison document, namely providing the complete amino acid protein containing natural Vitamin E and kernel fat product. And the comparison document is not involved with the words elaborating the features and production methods. According to the content of the search report, "Abelmoschus manihot Medicus kernel product" on the first line of the original Claim 1 is amended as "natural complete protein" and the replacement sheet is handed in for the amendment.
